## Europäisches Patentamt

## European Patent Office

## Office européen des brevets

(19)

(11) Publication number: **0 105 591**
**B1**

(12) **EUROPEAN PATENT SPECIFICATION**

(45) Date of publication of patent specification: **16.07.86**

(21) Application number: **83304852.3**

(22) Date of filing: **23.08.83**

(51) Int. Cl.⁴: $C\ 07\ C\ 1/20$, $B\ 01\ J\ 29/28$, $B\ 01\ J\ 29/36$

(54) **Catalytic conversion of methanol into light olefins.**

(30) Priority: **30.09.82 US 429933**

(43) Date of publication of application:
**18.04.84 Bulletin 84/16**

(45) Publication of the grant of the patent:
**16.07.86 Bulletin 86/29**

(84) Designated Contracting States:
**BE DE FR GB IT NL**

(56) References cited:
**EP-A-0 009 894**
**EP-A-0 060 103**
**US-A-4 025 575**
**US-A-4 049 573**
**US-A-4 247 731**
**US-A-4 349 461**

(73) Proprietor: **MOBIL OIL CORPORATION**
**150 East 42nd Street**
**New York New York 10017 (US)**

(72) Inventor: **Lee, Carol Sana**
**236 Cherry Hill Road**
**Princeton New Jersey 08540 (US)**
Inventor: **Stead, George Edward**
**14 Dawson Road**
**Kendall Park New Jersey 08824 (US)**

(74) Representative: **West, Alan Harry**
**Mobil Court 3 Clements Inn**
**London WC2A 2EB (GB)**

Courier Press, Leamington Spa, England.

## Description

This invention relates to a process for converting methanol into light olefins over a modified ZSM-12 zeolite.

A remarkable growth in the production of synthetic fibers, plastics and rubber has taken place in recent decades. Such growth, has, to a large extent, been supported and encouraged by an expanding supply of inexpensive petroleum-derived raw materials such as ethylene and propylene. However, increasing demand for these light olefins has, from time to time, led to periods of shortage, either due to a diminished supply of suitable feedstocks or to limited processing capacity. In any event, it is now considered highly desirable to provide efficient means for converting raw materials other than petroleum into light olefins.

One such non-petroleum source of light olefins is coal-derived methanol. In this respect, it is known that methanol can be catalytically converted into olefin-containing hydrocarbon mixtures by contact under certain conditions with particular types of crystalline zeolites. U.S. Patents 4,025,575 and 4,083,889, for example, both describe processes whereby methanol and/or dimethyl ether can be converted into an olefin-containing product over a ZSM-5 type (Constraint Index 1-12) zeolite catalyst. ZSM-5, in fact, converts methanol and/or dimethyl ether into hydrocarbons containing a relatively high concentration of light ($C_2$ and $C_3$) olefins.

Modification of zeolite methanol-conversion catalysts with, for example, silica, phosphorus, metal ions and metal oxides, can enhance selectivity of the methanol-conversion reaction for production of light olefins. For example, U.S. Patents 4,247,731 and 4,296,266 describe modification of zeolites such as Zeolite X and amorphous aluminum silicates with manganese and optionally with additional materials such as magnesium in order to enhance the ethylene selectivity of such zeolites when they are used to catalyze methanol/dimethyl ether conversion. U.S. Patent 4,049,573 describes the modification of ZSM-5 type zeolites with, for example, manganese, for a similar purpose. However, these prior documents reveal that, when such modified zeolites are used, ethylene is always the predominant hydrocarbon product except in those cases in which considerable quantities of aromatics are produced too, when significant amounts of propylene may be obtained.

It is also known that the production of light olefins, especially ethylene, by catalytic conversion of methanol can be optimized by varying one or more reaction parameters. Variations in reaction temperature, pressure and reactant space velocity can, for example, alter the selectivity of the methanol conversion reaction to produce different kinds of hydrocarbon products, for example, to maximize light olefin production.

Similarly, utilization of dilute methanol feed or inert diluents tends to increase selectivity towards ethylene and light olefin production. Notwithstanding the existence of processes suitable for converting methanol into high yields of light olefins, there is a continuing need to develop additional catalytic procedures suitable for converting an organic charge comprising methanol into light olefin products with improved light olefin selectivity and especially with improved selectivity to propylene, which, like ethylene, is a valuable material.

The present invention seeks to provide a process for converting a methanol feed into olefin-containing products with high selectivity to propylene.

According to the invention, there is provided a process for converting a methanol-containing feed into an olefin-containing hydrocarbon product rich in propylene, which comprises contacting the methanol-containing feed under conversion conditions with a ZSM-12 zeolite-containing catalyst characterized in that the catalyst contains a minor amount of magnesium and/or manganese.

In accordance with the invention, a methanol-containing feed is catalytically converted into an olefin-containing hydrocarbon product. The methanol-containing feed will, of course, contain methanol but may also contain dimethyl ether and/or additional components such as water or other diluents. Since methanol is miscible with water, the methanol may contain relatively large amounts of water.

The methanol-containing feed should, however, contain at least 60 wt.% methanol. Substantially pure methanol, such as industrial grade anhydrous methanol, is eminently suitable, although crude methanol, which usually contains from 12 to 20 wt.% water, or more dilute solutions, also may be used.

Small amounts of impurities such as higher alcohols, aldehydes, or other oxygenated compounds in the methanol-containing feed have little effect on the process of the invention. The methanol-containing feed may include dimethyl ether. In fact, the feed to the process may be up to 100% dimethyl ether but it is preferred that dimethyl ether constitutes not more than 20 wt.% of the total feed. Such amounts of dimethyl ether as may be formed in the process may, however, be recovered and recycled with fresh methanol feed, and the dimethyl ether content calculated on the total of recycle and fresh feed preferably will not exceed 20 wt.%.

According to one aspect of the invention, the feed to the process comprises only the methanol and associated organic oxygenates. According to another, preferred, aspect, the selectivity of methanol conversion for the production of light ($C_2$—$C_3$) olefins is increased by contacting the methanol feed with the catalyst described in detail below, in the presence of up to 20 mols, and preferably from 1 to 10 mols, of steam per mol of methanol feed. The steam may be provided directly by injecting the required amount of water or steam into the reaction zone; alternatively, steam may be provided totally or in part by water

2

present in the methanol-containing feed in a molar ratio of water to methanol of up to 20:1, preferably from 1:1 to 10:1. That water in the feed forms steam in the reaction zone under the process conditions.

According to the invention, the methanol-containing feed is catalytically converted into a light olefin-containing hydrocarbon product in which propylene is the major light olefin by contact with a modified catalyst comprising zeolite ZSM-12.

ZSM-12 is described in detail in U.S. Patent 3,832,449 and is characterized by its X-ray diffraction pattern as set out in that U.S. Patent. The ZSM-12 zeolite used in the process of the invention preferably has a crystal size from 0.02 to 0.5 micron.

The ZSM-12 zeolite-containing catalyst used in the process of the invention is preferably characterized by an activity, in terms of alpha value, from 25 to 250, and preferably from 50 to 150. The alpha value reflects the relative activity of the catalyst with respect to a high activity silica-alumina cracking catalyst. The method of determining alpha values is described in Journal of Catalysis, Vol. VI, Pages 278—287, 1966.

In the process of the invention, the ZSM-12 zeolite is preferably incorporated in a matrix comprising a material resistant to the temperature and other conditions employed in the process. Such matrix materials are useful as binders and impart greater resistance to the catalyst for the temperature, pressure and reactant feed stream velocity conditions which may be encountered in the process.

Useful matrix materials include both synthetic and naturally occurring substances, as well as inorganic materials such as clay, silica and/or metal oxides, and are well known in the art. The relative proportions of zeolite component and matrix material, on an anhydrous basis, may be from 1 to 99 percent by weight and more usually from 5 to 80 percent by weight of zeolite, based on the dry composite.

The present invention is based on the observation that modification of ZSM-12 zeolite catalysts with magnesium and/or manganese can enhance the selectivity of such catalysts even further to the production of $C_2$—$C_4$ light olefins, and especially to the production of propylene, during conversion of methanol and/or dimethyl ether into hydrocarbons.

The ZSM-12 zeolite-containing catalysts are modified by incorporation of a minor proportion of magnesium and/or a minor proportion of manganese. The modified zeolite composites can be prepared by contacting the ZSM-12 zeolite-containing composition with one or more compounds or complexes of magnesium and/or manganese and then preferably heating the composite to convert the modifying element(s) into oxide form. Incorporation can occur by the mechanisms of ion exchange or "stuffing" i.e. adsorption and/or impregnation. It should be emphasized that, while ion exchange can be used to incorporate the modifying metals into the zeolite, ion exchange alone will generally not provide the necessary amount or form preferred (i.e. the oxide form) of incorporated magnesium and manganese onto the zeolite catalyst.

Generally, the zeolite composite can be modified by contacting the composite with solutions of appropriate compounds of magnesium and/or manganese. Such solutions may be formulated from any suitable solvent which is inert to the metal-containing compound and the zeolite, including for example water, aromatic and aliphatic hydrocarbons, alcohols, and organic acids (such as acetic acid, formic acid and propionic acid) halogenated hydrocarbons, ketones and ethers. Generally, the most useful solvent will be water.

Suitable magnesium-containing compounds include the acetate, nitrate, benzoate, propionate, 2-ethylhexanoate, carbonate, formate, oxalate, amide, bromide, hydride, lactate, laurate, oleate, palmitate, salicylate, stearate and sulfide.

Suitable manganese-containing compounds include the acetate, nitrate, lactate, oxalate, carbonate, citrate, tartrate, bromide, chloride, sulfate and sulfide.

The amount of modifying metal incorporated onto the zeolite composition will, of course, depend upon several factors, for example reaction time, reaction temperature, concentration of the treating compound in the reaction mixture, the degree to which the zeolite composition has been dried prior to reaction with the metal-containing compounds, the conditions of drying of the zeolite composition after reaction with the treating compounds, and the amount and type of binder incorporated with the zeolite composition.

After the modifying magnesium and/or manganese compounds have been incorporated into the zeolite composite to the extent desired, the metal-containing composite is heated in the presence of oxygen, for example, in air. Although heating may be carried out at a temperature of about 150°C, higher temperatures, for example up to about 500°C, are preferred. Heating is generally carried out for 1—5 hours but may be extended to 24 hours or longer. While heating temperatures above about 500°C may be employed, they are generally not necessary. After the metal-containing composite is heated in air at elevated temperatures, the modifying metals are actually present at least in part in the zeolite composite in an oxidized state, such as MgO and MnO.

Generally, the modifying metal oxides are incorporated in amounts from 0.1% to 10% by weight of the zeolite composite, preferably from 1% to 10% by weight of the zeolite composite, calculated on the elemental metals. Magnesium oxide is advantageously incorporated in an amount from 0.1% to 10%, more preferably from 0.1% to 5%, by weight, calculated as magnesium. Manganese oxide is advantageously incorporated in an amount from 0.1% to 10%, more preferably from 1% to 10%, by weight, calculated as manganese.

In accordance with the invention, a feed comprising methanol, dimethyl ether, methanol/dimethyl ether mixtures or mixtures of such organic materials with water is contacted in the vapor phase with the magnesium and/or manganese modified ZSM-12 catalyst materials under reaction conditions suitable for effecting conversion of methanol and/or dimethyl ether into olefins. Such conditions include an operating temperature from 260°C to 540°C, preferably 400°C to 450°C; a pressure from 10 kPa to 3000 kPa, preferably 500 to 1000 kPa; and a weight hourly space velocity (WHSV) of the organic reactants from 0.1 to 30, preferably 1 to 10. Carrier gases or diluents may be injected into the reaction zone, for example hydrogen, nitrogen, helium, water, carbon monoxide, carbon dioxide, or mixtures of these gases.

When water is employed along with the organic feed, the amount of water is generally at least 0.25 moles of water per mole of organic reactant. Preferably, the amount of water added is greater than 0.5 moles of water per mole of organic reactant. The amount of water initially added to the organic reactant will not usually exceed 40 moles per mole of organic reactant. Water in these amounts can generally be passed to the reaction zone at a WHSV from 1 to 20, more preferably from 2 to 20.

The reaction conditions, including the presence or absence of diluents, can affect the selectivity of the process to light olefin and propylene production as well as percent conversion of organic reactants to hydrocarbon product. However, it has been discovered that for a given set of reaction conditions, the magnesium and/or manganese-modified ZSM-12 catalysts provide improved methanol conversion and/or propylene selectivity in comparison with similar processes employing other zeolites.

The process may be carried out as a batch-type, semi-continuous or continuous operation utilizing a fixed, fluidized or moving bed catalyst system. A preferred process entails use of a catalyst zone in which the alcohol or ether charge optionally together with added water is passed concurrently or countercurrently through a fluidized or moving bed of particle-form catlyst. The latter, after use may be conducted to a regeneration zone where coke is burned from the catalyst in an oxygen-containing atmosphere, for example air, at an elevated temperature, after which the regenerated catalyst is recycled to the conversion zone for further contact with the methanol- and/or ether-containing feed.

The product stream of the process contains steam and a hydrocarbon mixture of paraffins and olefins, substantially devoid of aromatics. The mixture is particularly rich in light olefins, and especially rich in propylene. Thus, the predominant hydrocarbon product constitutes valuable petrochemical. The steam and hydrocarbon products may be separated from one another by methods well known in the art. Preferably, the unconverted methanol and/or dimethyl ether, as well as at least part of the water in the product, are recycled to the reaction zone.

The following Examples illustrate the invention.

Example I

Zeolite ZSM-12 is prepared in the following manner: five grams of sodium aluminate (41.8% $Al_2O_3$) are dissolved in a solution of 96.2 grams of tetraethylammoniumbromide and 17.5 grams of NaOH in 300 grams $H_2O$. Five hundred grams of colloidal silica, e.g., Ludox, are added to the solution and mixed for 15 minutes. The resulting mixture is crystallized in a polypropylene jar without agitation for 84 days at 100°C. Analysis by X-ray diffraction identifies the product as ZSM-12 with a $SiO_2/Al_2O_3$ ratio of 82. The product is, washed and dried at 110°C. The dried powder is pre-calcined for 3 hours at 370°C in air and then cooled to room temperature in a desiccator. This is followed by four contacts each with 10 ml of 5 percent $NH_4Cl$/gram of calcined powder at 90°C for 1 hour. Each contact is made with stirring. The product is washed free of $Cl^-$ ions at room temperature and then dried at 110°C. The sodium content of the zeolite at this stage is less than 0.01 percent. The dried powder is then calcined by heating it in air at 538°C for 10 hours, and then cooled in a desiccator.

Zeolite ZSM-12 made in this general manner can be synthesized with various silica to alumina ratios and can be pretreated, e.g. by steaming, to produce zeolites of various alpha values.

Example II

ZSM-12 samples, prepared in the general manner described in Example I, are modified in their ammonium exchanged form to incorporate both magnesium and manganese. Composites of $NH_4$-ZSM-12 in 35% alumina binder are washed twice with an aqueous solution of 1M $Mn(OAc)_2$ and 0.2M $Mg(OAc)_2$, for two hours at reflux, followed by water washes, drying and calcination in air at 500°C. Samples prepared in this manner contain 4.2% Mn and 0.5% Mg by weight. Catalyst samples containing only magnesium and catalyst samples containing only manganese are also made in similar manner using either a 1 M solution of magnesium acetate or a 1 M solution of manganese acetate. Mg-modified zeolites prepared in this manner contain 3.0% Mg by weight and Mn-modified zeolites prepared in this manner contain about 3.9% Mn by weight.

Example III

Using catalyst samples prepared in the general manner of Examples I and II, methanol/water mixtures are catalytically converted into hydrocarbons and the resulting hydrocarbon products are analyzed. In such a procedure, a fixed bed, down-flow quartz reactor with a central thermowell is used for atmospheric pressure runs. Liquids are fed with a syringe pump. Gas is metered with a manual low flow controller. A 12.7 mm stainless steel reactor with central thermowell is used in the down-flow mode for pressure runs. 1.5 grams of catalyst are used in the reactors and products are collected for one hour periods. Gases are measured in a dry gas tower with a mercury-sealed float, and analyzed on a silica gel column with a thermoconductivity detector. Liquids are condensed with ice-water cooling. Methanol/dimethyl ether conversions are calculated on a carbon molar basis (approximately equivalent to a $CH_2$ basis).

Methanol conversion runs are made using several types of ZSM-12 zeolite catalysts. Catalyst description, reaction conditions and methanol conversion results are set forth in Table I.

TABLE I

Methanol conversion over ZSM-12 effect of cation and short term aging effects

| Run No. | 1 | 2 | 3 | 4 | 5 |
|---|---|---|---|---|---|
| Catalyst | HZSM-12 | HZSM-12 | MgZSM-12 | Mn-ZSM-12 | Mg/Mn-ZSM-12 |
| $SiO_2$:$Al_2O_3$/$\alpha$ | (180/103) | (180/103) | (180/20) | (180/—) | (180/13) |
| WHSV    MeOH | 2.1 | 2.1 | 2.1 | 2.1 | 2.1 |
| $H_2O$ | 3.5 | 3.5 | 3.5 | 3.5 | 3.5 |
| Temperature (°C) | 390 | 450 | 450 | 450 | 450 |
| Time on stream (hrs) | — | — | — | — | 2 |
| Conversion to hydrocarbons (C mol.%) | 97.3 | 99.5 | 80.5 | 93 | 91.9 |
| Selectivity to hydrocarbons (wt.%) | | | | | |
| COX | 0.1 | 0.1 | 0.1 | 0 | 0 |
| $C_1$ | 0.4 | 0.5 | 0.4 | 0.4 | 0.5 |
| $C_2^\circ$ | 0.1 | 0.1 | 0.1 | 0.1 | 0.1 |
| $C_2=$ | 3.2 | 6.4 | 3.1 | 3.5 | 3.8 |
| $C_3^\circ$ | 5.2 | 5.4 | 1.5 | 2.4 | 2.5 |
| $C_3=$ | 20.0 | 35.1 | 48.3 | 42.0 | 59.5 |
| $C_4^\circ$ | 18.6 | 13.5 | 4.6 | 7.9 | 6.1 |
| $C_4=$ | 16.2 | 20.9 | 17.6 | 17.8 | 16.9 |
| $C_5$ | 2.0 | 3.4 | 10.4 | 13.6 | 1.2 |
| $C_6$ | 0 | 0.2 | 0.9 | 1.6 | 0 |
| liquid | 34.3 | 14.4 | 12.9 | 10.6 | 9.6 |
| $C_2$—$C_4=$ | 39.4 | 62.4 | 69.0 | 63.3 | 80.2 |

The Table I data demonstrate that methanol conversion over a ZSM-12 zeolite catalyst modified by incorporation of both magnesium and manganese is especially selective to light olefin production with the major light olefin product being propylene.

Example IV

Using the general testing procedures described in Example III, additional conversion runs are made at various reaction temperatures using the Mg/Mn-ZSM-12 zeolite sample employed in Example III. Reaction conditions and conversion results are set forth in Table II.

TABLE II

Methanol conversion over Mg/Mn-ZSM-12 effect of temperature

| Run No. | | 1 | 2 | 3 | 4 | 5 | 6 |
|---|---|---|---|---|---|---|---|
| WHSV | MeOH | 2.1 | 2.1 | 2.1 | 2.1 | 2.1 | 2.1 |
| | $H_2O$ | 3.5 | 3.5 | 3.5 | 3.5 | 3.5 | 3.5 |
| Temperature (°C) | | 350 | 370 | 390 | 420 | 450 | 475 |
| Conversion to hydrocarbons (C mol. %) | | 32.9 | 43.3 | 65.5 | 90.0 | 99.2 | 100 |
| Selectivity to hydrocarbons (wt.%) | | | | | | | |
| COX | | 0 | 0 | 0 | 0 | 0 | 0 |
| $C_1$ | | 0.5 | 0.5 | 0.4 | 0.3 | 0.4 | 0.5 |
| $C_2°$ | | 0 | 0 | 0 | 0 | 0.1 | 0.1 |
| $C_2=$ | | 0.7 | 0.7 | 1.1 | 2.1 | 3.7 | 5.3 |
| $C_3°$ | | 1.1 | 1.0 | 1.4 | 1.8 | 2.1 | 2.1 |
| $C_3=$ | | 39.3 | 34.5 | 40.2 | 45.4 | 49.6 | 52.1 |
| $C_4°$ | | 10.4 | 9.0 | 9.5 | 7.9 | 5.8 | 4.7 |
| $C_4=$ | | 18.7 | 20.5 | 19.0 | 19.0 | 18.3 | 18.6 |
| $C_5$ | | 16.4 | 13.7 | 13.9 | 12.8 | 11.8 | 11.4 |
| $C_6$ | | 1.4 | 1.5 | 1.8 | 1.7 | 1.5 | 1.4 |
| liquid | | 11.4 | 18.4 | 12.8 | 8.7 | 6.6 | 3.6 |
| $C_2—C_4=$ | | 58.8 | 55.8 | 60.3 | 66.5 | 71.6 | 76.0 |

The Table II data demonstrate that, at higher temperatures, Mg/Mn-ZSM-12 promotes greater conversion of methanol into hydrocarbons with generally higher selectivity to production of $C_2—C_4$ olefins. Propylene is the major light olefin product at all temperatures.

Example V

Using the general testing procedures described in Example III, additional methanol conversion runs are made over Mg/Mn-ZSM-12 using different temperatures, different space velocities for methanol and water and, in one instance, using a nitrogen diluent. Reaction conditions and conversion results are set forth in Table III.

7

TABLE III
Methanol conversion over Mg/Mn-ZSM-12 effect of dilution

| Run No. | | 1 | 2 | 3 | 4 | 5 |
|---|---|---|---|---|---|---|
| WHSV | MeOH | 2.2 | 2.1 | 2.1 | 2.2 | 2.2 |
| | $H_2O$ | — | 3.5 | 3.5 | 6.2 | — |
| | $N_2$ | — | — | — | — | 2.8 |
| Temperature (°C) | | 362 | 362 | 450 | 460 | 450 |
| Conversion to hydro-carbons (C mol.%) | | 64.6 | 19.9 | 99.2 | 99.2 | 98.6 |
| Selectivity to hydrocarbons (wt.%) COX | | — | — | 0 | 0 | 0 |
| $C_1$ | | 0.6 | 0 | 0.4 | 0.4 | 0.7 |
| $C_2°$ | | 0 | 0 | 0.1 | 0 | 0 |
| $C_2=$ | | 1.6 | 1.0 | 3.7 | 3.6 | 1.5 |
| $C_3°$ | | 1.3 | 0.9 | 2.1 | 1.3 | 1.4 |
| $C_3=$ | | 17.4 | 28.7 | 49.6 | 55.1 | 44.4 |
| $C_4°$ | | 14.5 | 7.8 | 5.8 | 3.8 | 7.3 |
| $C_4=$ | | 15.2 | 26.0 | 18.3 | 18.9 | 22.0 |
| $C_5$ | | 28.9 | 25.8 | 11.8 | 11.3 | 18.6 |
| $C_6$ | | 28.9 | 25.8 | 1.5 | 1.8 | 2.0 |
| liquid | | 20.5 | 9 | 6.6 | 3.6 | 0 |
| $C_2$—$C_4=$ | | 34.2 | 55.7 | 71.6 | 77.6 | 67.9 |

The Table III data demonstrate the selectivity to light olefins in general and to propylene in particular for methanol conversion over Mg/Mn-ZSM-12.

**Claims**

1. A process for converting a methanol-containing feed into an olefin-containing hydrocarbon product in which propylene is the major light olefin which comprises contacting the methanol-containing feed under conversion conditions with a ZSM-12 zeolite-containing catalyst characterized in that the catalyst contains a minor amount of magnesium and/or manganese.

2. A process according to Claim 1, wherein the magnesium and/or manganese are present in the catalyst at least in part in the form of their oxides.

3. A process according to Claim 1 or Claim 2, wherein the catalyst contains from 0.1% to 10% by weight magnesium.

4. A process according to any one of Claims 1 to 3, wherein the catalyst contains from 0.1% to 10% by weight manganese.

5. A process according to any one of Claims 1 to 4, wherein the conversion conditions include a reaction temperature from 400°C to 450°C and a pressure from 500 to 1000 kPa.

6. A process according to any one of Claims 1 to 5, wherein the conversion conditions include a weight hourly space velocity for the organic reactants of the methanol-containing feed from 0.1 to 30.

7. A process according to any one of Claims 1 to 6, wherein the methanol-containing feed contains water in an amount from 1 to 20 moles of water per mole of organic reactants.

8. A process according to Claim 7, wherein the weight hourly space velocity of the water is from 1 to 20.

**Patentansprüche**

1. Verfahren zur Umwandlung einer Methanol enthaltenden Zufuhr in ein Olefin enthaltendes Kohlenwasserstoffprodukt, in dem Propylen die Hauptkomponente der leichten Olefine bildet, welches das Inkontaktbringen der Methanol enthaltenden Zufuhr unter Umwandlungsbedingungen mit einem ZSM-12-Zeolith enthaltenden Katalysator umfaßt, dadurch gekennzeichnet, daß der Katalysator eine geringe Menge an Magnesium und/oder Mangan umfaßt.

2. Verfahren nach Anspruch 1, worin Magnesium und/oder Mangan in den Katalysator zumindest teilweise in Form ihrer Oxide vorhanden sind.

3. Verfahren nach Anspruch 1 oder 2, worin der Katalysator von 0,1 bis 10 Gew.-% Magnesium enthält.

4. Verfahren nach einem der Ansprüche 1 bis 3, worin der Katalysator von 0,1 bis 10 Gew.-% Mangan enthält.

5. Verfahren nach einem der Ansprüche 1 bis 4, worin die Umwandlungsbedingungen eine Reaktionstemperatur von 400 bis 450°C und einen Druck von 500 bis 1000 kPa umfassen.

6. Verfahren nach einem der Ansprüche 1 bis 5, worin die Umwandlungsbedingungen eine stündliche Gewichts-Raum-Geschwindigkeit für die organischen Reaktanten der Methanol enthaltenden Zufuhr von 0,1 bis 30 umfassen.

7. Verfahren nach einem der Ansprüche 1 bis 6, worin die Methanol enthaltende Zufuhr Wasser in einer Menge von 1 bis 20 Mol Wasser pro Mol der organischen Reaktanten enthält.

8. Verfahren nach Anspruch 7, worin die stündliche Gewichts-Raum-Geschwindigkeit des Wassers von 1 bis 20 beträgt.

**Revendications**

1. Procédé de conversion d'une charge contenant du méthanol en hydrocarbures contenant des oléfines dans lesquelles le propylène est l'oléfine légère majeure, qui consiste à mettre en contact la charge contenant du méthanol dans les condition de conversion avec un catalyseur contenant une zéolite ZSM-12, caractérisé en ce que le catalyseur contient une quantité mineure de magnésium et/ou de manganèse.

2. Procédé selon la revendication 1, dans lequel le magnésium et/ou le manganèse sont présents dans le catalyseur au moins en partie sous forme d'oxydes.

3. Procédé selon la revendication 1 ou la revendication 2, dans lequel le catalyseur contient de 0,1 à 10% en poids de magnésium.

4. Procédé selon l'une quelconque des revendications 1 à 3, dans lequel le catalyseur contient de 0,1 à 10% en poids de manganèse.

5. Procédé selon l'une quelconque des revendications 1 à 4, dans lequel les conditions de conversion comprennent une température de réaction de 400°C à 450°C et une pression de 500 à 1 000 kPa.

6. Procédé selon l'une quelconque des revendications 1 à 5, dans lequel les conditions de conversion comprennent une vitesse spatiale horaire pondérale des réactifs organiques et de la charge contenant du méthanol de 0,1 à 30.

7. Procédé selon l'une quelconque des revendications 1 à 6, dans lequel la charge contenant du méthanol contient de l'eau en quantité variable entre 1 et 20 moles d'eau par mole de réactif organique.

8. Procédé selon la revendication 7, dans lequel la vitesse spatiale horaire pondérale de l'eau est comprise entre 1 et 20.